# EUROPEAN PATENT APPLICATION

(11) **EP 2 995 948 A1**
(43) Date of publication of application: **16.03.2016**
(21) Application number: 14184110.6
(22) Date of filing: 09.09.2014
(51) Int. Cl.: G01N 33/50, C12N 5/00, A61K 31/06, A61K 31/277, A61K 31/405, A61K 31/455, A61K 31/496

(54) **Methods and compounds useful in hematopoietic stem cell medicine**

(71) Applicant: Ecole Polytechnique Federale de Lausanne (EPFL), 1015 Lausanne (CH)
(72) Inventor: Vannini, Nicola, 1090 La Croix sur Lutry (CH); Lutolf, Matthias, 1131 Tolochenaz (CH); Auwerx, Johan, 1164 Buchillon (CH); Naveiras, Olaia, 1090 La Croix sur Lutry (CH); Girotra, Mukul, 1005 Lausanne (CH)
(74) Representative: reuteler & cie SA

(57) **Abstract**

The present invention relates to a specific marker for stem cells having long-term multi-lineage blood reconstitution capability and uses thereof for the preparation of cell composition in view of hematopoietic stem cell medicine and in particular regenerative therapies and bone marrow transplantation. Further, the invention relates to methods and compositions useful in the prevention and/or treatment of decreased blood cell levels and the associated risk of infection.

## Description

### Field of the Invention

The present invention pertains generally to the fields of hematopoietic stem cell medicine and in particular regenerative therapies and bone marrow transplantation. In particular, the invention relates to methods and compositions useful in the regeneration of damage human tissue, *ex vivo* propagation of stem/progenitor cells and in the treatment of blood or immune system diseases.

### Background of the Invention

Lifelong blood production relies on a finely orchestrated balance between hematopoietic stem cell (HSC) quiescence, self-renewal and differentiation. The mechanisms that control how the stem cells choose these disparate fates are unknown. The hematopoietic system has the capacity to generate 200 billion erythrocytes per day during the entire life of an organism. This incredible long-term cell production is orchestrated by HSCs that, at the same time, have to maintain their function throughout the life. The maintenance of the blood system is ensured by a pool of HSCs residing in hypoxic areas of the bone marrow (BM) (hypoxic niche). These rare cells are capable of lifelong self-renewal and commitment to multipotent progenitors (MPP).
Preservation of a functional HSC pool is achieved by maintaining a pool of quiescent HSCs, a strategy adopted by the cell to avoid cellular stress and damages (Wilson et al., 2008, Cell, 135(6), 118-29*).* Cellular metabolism plays a pivotal role in this process and recently has emerged as master regulator of HSC function (Tabuko et al., 2013, Cell Stem Cell, 12(1), 49-61*).* If modulation of cellular metabolism is crucial in quiescence maintenance it also directs HSC fate decisions *(*Ito et al., 2012, Nat. Med., 18 (9), 1350-8).
Transplantation of BM is a potentially life-saving treatment therapy for haematological malignancies such as leukemia and other diseases of the blood and immune system which include, but are not limited to, cancers (e.g., leukemia, lymphoma), blood disorders (e.g., inherited anemia, inborn errors of metabolism, aplastic anemia, beta-thalassemia, Blackfan-Diamond syndrome, globoid cell leukodystrophy, sickle cell anemia, severe combined immunodeficiency, X-linked lymphoproliferative syndrome, Wiskott-Aldrich syndrome, Hunter's syndrome, Hurler's syndrome Lesch Nyhan syndrome, osteopetrosis), chemotherapy rescue of the immune system, and other diseases (e.g., autoimmune diseases, diabetes, rheumatoid arthritis, system lupus erythromatosis).
In order to improve the outcome of post-transplanted patients it is crucial to reduce the risk of severe infections and bleeding by shortening the blood recovery period. For many decades, HSCs have been successfully used for the treatment of hematological and immune diseases, but their limited number hinders more reliable and broader applications of HSC-based therapies. In fact, if hematopoietic stem cells are by far the most extensively used stem cells for clinical applications, HSC therapy is still a very risky procedure, with associated mortality close to 50% when applied to hematological malignancies, mostly due to the lag of neutropenia subsequent to BM ablation.
Further, countless attempts to propagate functional HSCs *'ex vivo'* have failed, because long-term self-renewal and *in vivo* regenerative capacity is rapidly lost in culture. Whereas the progression of long-term HSCs, also called LT-HSCs (typically defined by the markers Lin- cKit+ Sca-1+ (LKS CD150+ CD34-) to short-term HSCs, also called ST-HSCs (LKS CD150+CD34+) and multipotent progenitors, also called MPPs (LKS CD150- CD34+) is mirrored by only small changes in the repertoire of cell surface markers and the surface marker repertoire does not reliably predict *in vivo* function. Clinical studies showed that *ex-vivo* expanded HSCs have only a transient contribution to hematopoiesis. In fact, expanded HSCs engraft rapidly but did not contribute to long-term multilineage engraftment, potentially by loss of self-renewal ability. The lack of reliable phenotypic or genetic markers to distinguish self-renewing, long-term HSCs from their earliest, functionally compromised progeny has made it difficult to elucidate the molecular mechanisms of self-renewal.

In human autologous and allogeneic bone marrow transplantations, currently used as therapies for diseases such as blood malignancies, a large number of patients does not find a histocompatible donor neither in the family nor in the world registry, and thus must recur to other sources of HSCs for transplantation such as cord blood. Unfortunately, cord blood (CB) contains a much lower absolute number of HSCs than mobilised peripheral blood CD34+ cells (MPBCs) or BM, making the CB less preferred for treatment use in adult patients. The ability to increase the number of hematopoietic stem and progenitor cells would in theory reduce the amount needed per MPBC or bone marrow donation or avoid the need for pooling hematopoietic stem cells from different umbilical cords (dual transplantation using multiple cord blood units) which requires increased stringency in human antigen leukocyte (HLA) matching.

However, human bone marrow expansion cultures have been shown to present limited hematopoietic potential, producing decreasing numbers of hematopoietic progenitor and mature blood cells with cell production ceasing by about six to eight weeks and in spite of many efforts to advance *in vitro* expansion using exogenous growth factors, only a limited increase in the number of pluripotent cells has been successfully achieved. In addition, it is currently very difficult to reliably expand long term repopulating (LT)-HSCs isolated from BM- and CB-HSCs, exacerbating the need for a new supply of LT-HSCs.

Nicotinamide adenine dinucleotide (NAD⁺) precursors have been already used in clinic in the form of nicotinic acid (NA) as treatment for high circulating levels of cholesterol and blood lipids (Karpe et al., 2004, Lancet, 363(9424), 1892-4*;* Canto et al., 2012, Cell. Metab., 15(6), 838-47*).* However, the effect of NAD levels on the hematopoietic system has not been extensively studied.

It derives that HSC therapy potentials would be considerably increased with the development on one hand of techniques to precisely distinguish stem cells having a HSC long-term multilineage blood reconstitution potential from cells which have lost long-term self-renewal capabilities and another hand of valuable strategies to stimulate blood production during the immediate post-transplant period to avoid severe infections and decrease mortality associated with HSC transplants.

Therefore, there are several needs for the development of strategies useful for clinical HSC expansion and therapeutic manipulation of HSC fate in order to enhance regeneration of the blood system, notably in cancer treatments.

### Summary of the Invention

The present invention is directed to the unexpected findings that low mitochondrial membrane potential (ΔΨm) is a robust marker for long-term multi-lineage blood reconstitution capability for freshly isolated as well as *in vitro*-cultured HSCs within each hematopoietic stem or early progenitor population. Quiescent and cycling HSCs, either under homeostatic conditions or in activated conditions where the number of cycling is increased, have comparable (ΔΨm) profiles. ΔΨm is therefore a marker for functional HSCs, independently of their cell cycle status. Further, the present invention is based on the unexpected findings that daughter cells retaining a low ΔΨm maintain stemness, whereas those that engage mitochondria to support ATP production are differentiated and that, strikingly, chemical uncoupling of the electron transport chain to disrupt ATP synthesis in the mitochondrion, while forcing the maintenance of a low mitochondrial membrane potential in HSC cells, instructs those cells to undergo self-renewal, even under culture conditions that normally induce rapid differentiation.
The present invention is directed to the other unexpected findings that the use of an agent reducing the mitochondrial potential such as agents uncoupling electron transport from ATP generation within the mitochondria or agents such as nicotinamide riboside (NR) or nicotinic acid (NA), are able to maintain or expand HSCs *ex vivo* and, when administered through the food, expand hematopoietic progenitor compartments in the mouse bone marrow and improve survival and blood recovery after transplantation, while raising the proportion of dividing HSCs cells with low ΔΨm. These findings are even more surprising since earlier studies showed that the administration of nicotinamide did not increase the platelet levels in mice (Konieczna et al., 2013, Blood cells, molecules & diseases, 50, 171-6*).*

A first aspect of the invention provides a method for *ex-vivo* hematopoietic stem cell expansion.
A second aspect of the invention provides a method for enriching hematopoietic stem cell preparations in functional stem cells having long-term multi-lineage blood reconstitution capability.
A third aspect of the invention provides a method of *ex-vivo* maintaining and/or extending stemness of hematopoietic stem cell population.
A fourth aspect of the invention provides a kit for selecting functional stem cells having long-term multi-lineage blood reconstitution capability.
A fifth aspect of the invention provides a kit or a cell expansion culture medium for hematopoietic stem cell comprising at least one mitochondrial membrane potential reducing agent.
A sixth aspect of the invention provides a mitochondrial membrane potential reducing agent for use in the prevention and/or treatment of a decreased blood cell level as compared to a control blood cell level, such as severe neutropenia and/or severe thrombocytopenia such as found in hematopoietic stem cell post-transplanted subjects or subjects undergoing ablative chemotherapy for solid tumors or suffering from severe immunological disorders.
A seventh aspect of the invention provides a use of a mitochondrial membrane potential reducing agent for the preparation of a medicament or a food supplement for prevention and/or treatment of a decreased blood cell level as compared to a control blood cell level.
An eighth aspect of the invention provides a composition comprising mitochondrial membrane potential reducing agent and an agent useful in the prevention and/or treatment of decreased blood cell level such as G-CSF analogues (i.e. filgrastim) or TPO receptor analogues (i.e. N-PLATE).
A ninth aspect of the invention provides a method of preventing and/or treating of a decreased blood cell level as compared to a control blood cell level in a subject, said method comprising administering an effective amount of a mitochondrial membrane potential reducing agent in a subject by oral administration, injection or as a food supplement. Such mitochondrial membrane potential reducing agent treatment can be combined with current standard of therapy, namely G-CSF analogues (i.e. filgrastim) for neutropenia and TPO receptor analogues (i.e. N-PLATE) for autoimmune thrombocytopenia.
A tenth aspect of the invention provides a method for promoting standard blood profile recovery and/ or preventing, or attenuating the risk of infection in hematopoietic cell-depleted patients, said method comprising administering an effective amount of a mitochondrial membrane potential reducing agent in a subject by injection or as a food supplement.

### Description of the figures

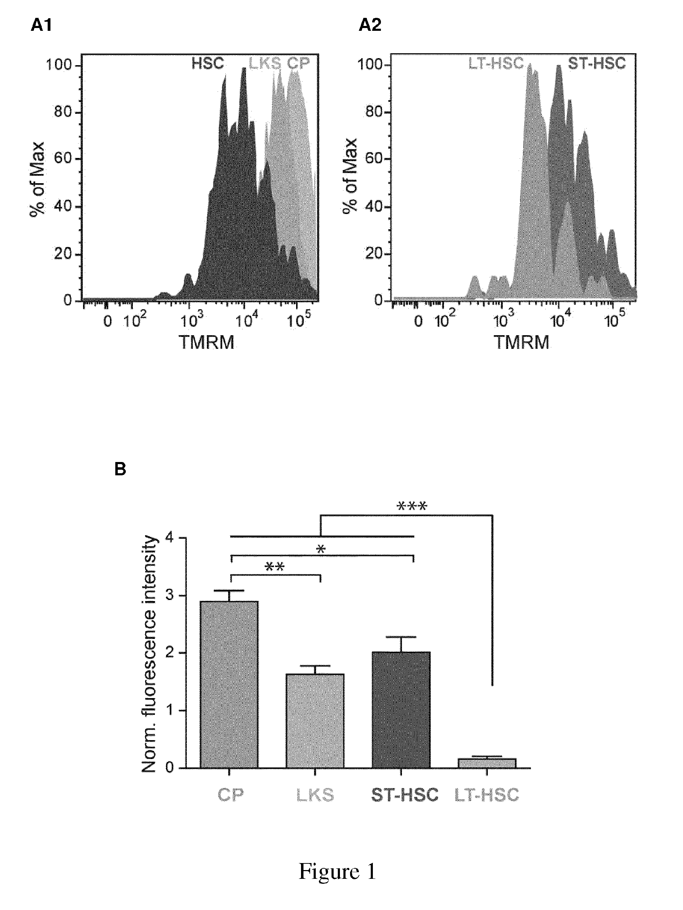

**Figure 1** shows distinct mitochondrial membrane potential (ΔΨm) for phenotypically defined hematopoietic stem and progenitor cell populations as measured by TMRM fluorescence as described in Example 1. **A1 and A2:** Flow cytometry analysis of CPs, LKS, ST-HSC and LT-HSC based on (ΔΨm) labelled with TMRM. Each population is marked by a differential ΔΨm level with a stepwise increase from the most primitive to the most committed population; **B:** Confocal image analysis of live TMRM-labelled stem/progenitor cells confirms an increase of ΔΨm as represented by normalized flurorescence intensity with increasing commitment level (n=7). Three representative examples are shown for each cell population. ***P < 0.001, **P < 0.01 and *P < 0.05.

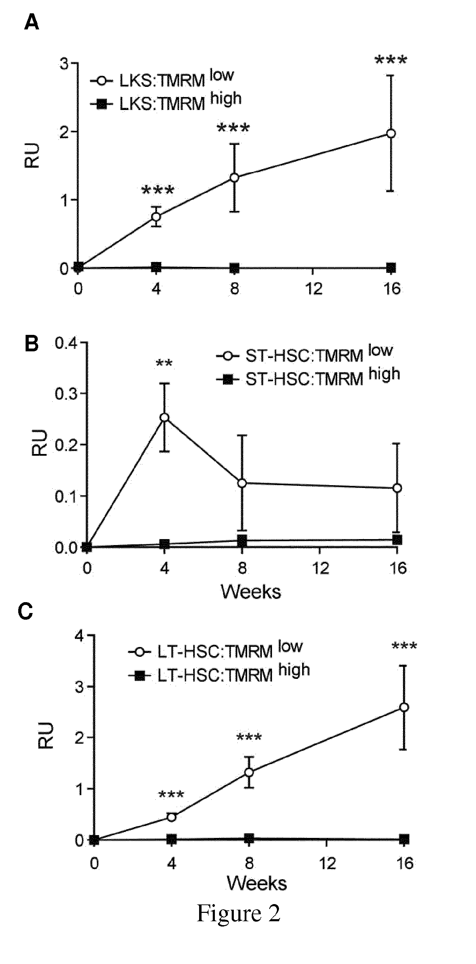

**Figure 2** shows that engraftment capability (multi-lineage reconstitution capacity), as measured by repopulation unit (RU) as tested in competitive transplantation assay, as described in Example 1, is restricted to HSC subpopulations with low ΔΨm exclusively.
**A:** LKS population, containing all multipotent stem and progenitor cells in the bone marrow, long-term stemness is restricted to TMRM^{low} cells (LKS:TMRM^{low}) (n=8 for each condition). **B:** In the phenotypically defined ST-HSC compartment, stemness is restricted to TMRM^{low} cells (ST-HSC:TMRM^{low}) (n=9 for each condition). C: In the phenotypically defined LT-HSC compartment, stemness is restricted to TMRM^{low} cells (LT-HSC:TMRM^{low}) (n=9 for each condition). ***P < 0.001, **P < 0.01 and *P < 0.05.

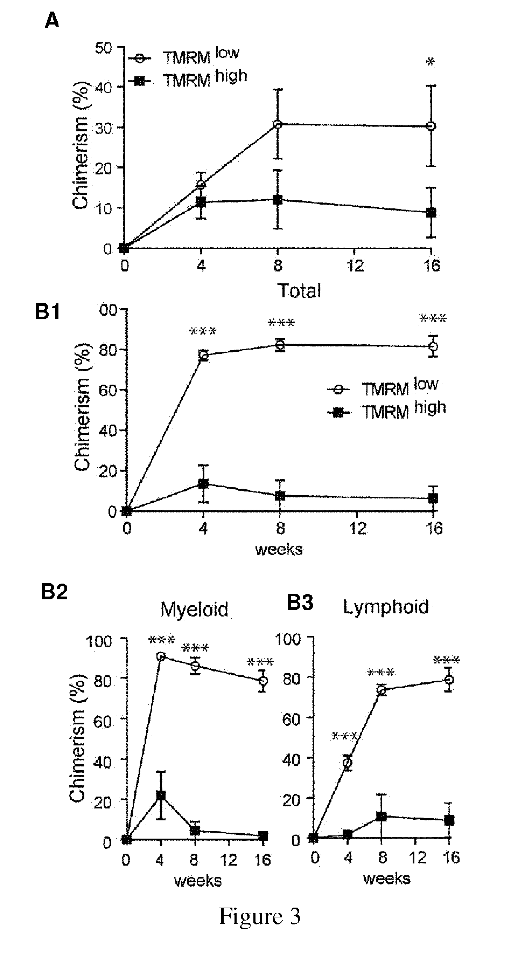

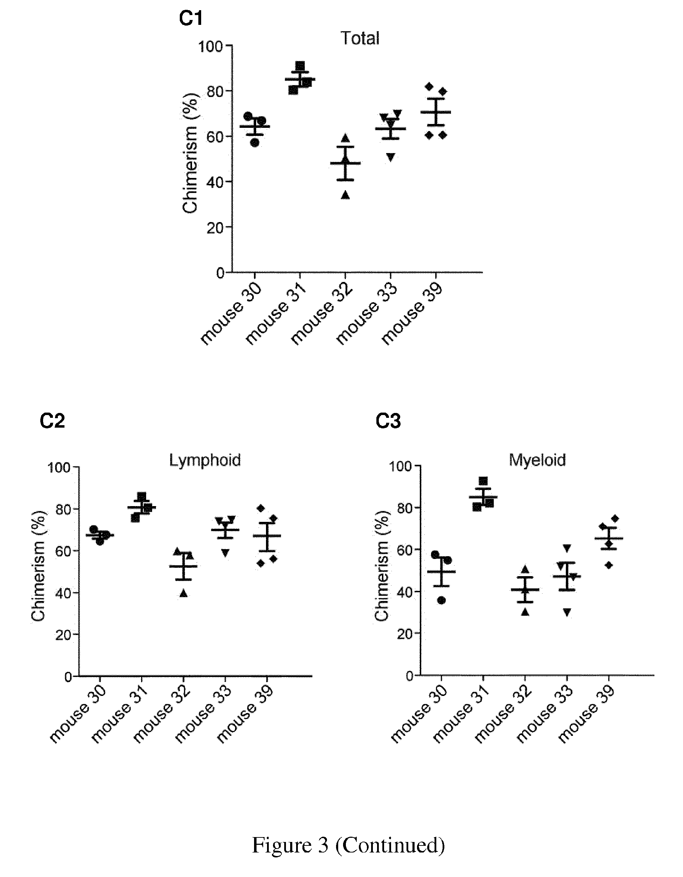

**Figure 3** shows that a low ΔΨm marks self-renewing HSCs in culture measured by the percentage of chimerism as tested in Example 3. **A:** Percentages of chimerism of TMRM^{low} cell and of TMRM^{high} cells fractions of culture-expanded HSC progeny after 5-day *in vitro* expansion. **B:** Percentages of chimerism of TMRM^{low} fraction of the first generation of daughter cells (i.e. dividing one time) compared to TMRM^{high} cells, supporting extremely higher long-term multi-lineage blood reconstitution efficiency for TMRM^{low} fraction and providing evidence for self-renewing versus differentiating HSC divisions in culture (n=10 for each condition) for total blood (**B1**) as well as the lymphoid **(B2)** and myeloid lineages **(B2); C:** Secondary transplantation of BM derived from TMRM^{low} injected primary recipient mice show long-term reconstitution at 8 weeks as shown by their percentages of chimerism for total blood (**C1**), lymphoid **(C2)** and myeloid lineages **(C2).** ***P < 0.001, **P < 0.01 and *P < 0.05.

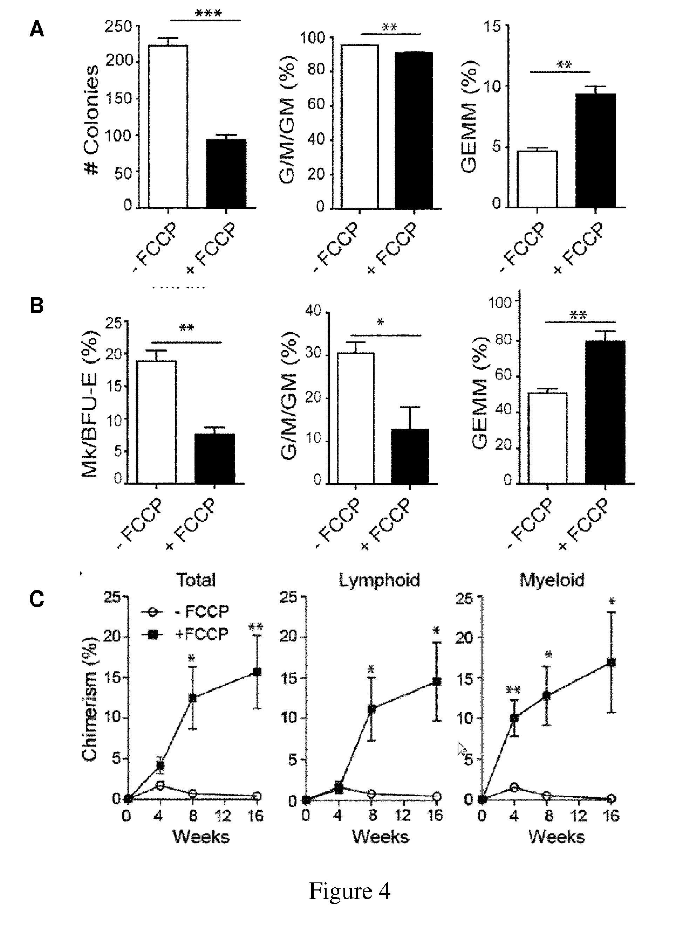

**Figure 4** shows that the modulation of mitochondrial metabolism alters HSC fate as measured in short- and long-term colony forming unit (CFU) assays in presence or absence of electron transport chain uncoupling agent (FCCP) as described in Example 3. **A:** Total number of colonies and percentage of colonies derived from the most committed progenitors (macrophage colonies, M, granulocyte/macrophage colonies, GM) (left and middle panel, respectively) and derived from the most primitive progenitor cells (right panel) on total bone marrow cells; **B:** Number and percentage of colonies derived from the most committed progenitors (mk, G, M and GM) (left and middle panel) and derived from the most primitive progenitor cells (right panel) on phenotypic LT-HSC:TMRM^{low} cells. **C:** Levels of multi-lineage reconstitution measured as percentage of Chimerism in cells cultured in presence of FCCP in total cells (left), lymphoid cells (middle panel and myeloid cells (right panel)). ***P < 0.001, **P < 0.01 and *P < 0.05. (CFU-)G: Colony forming unit-granulocyte; (CFU-)M: Colony forming unit-macrophage; (CFU-)GM: Colony forming unit-granulocyte, macrophage; (CFU-)GEMM: Colony forming unit-granulocyte, erythrocyte, macrophage, megakaryocyte); Mk/BFU-E: Megakaryocyte/Burst forming unit-erythroid.

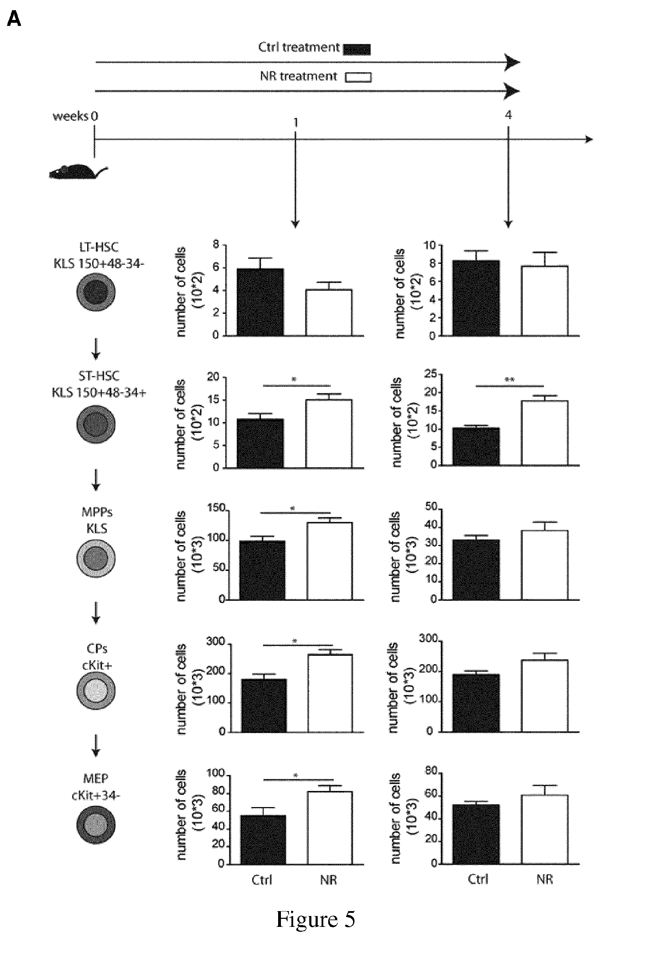

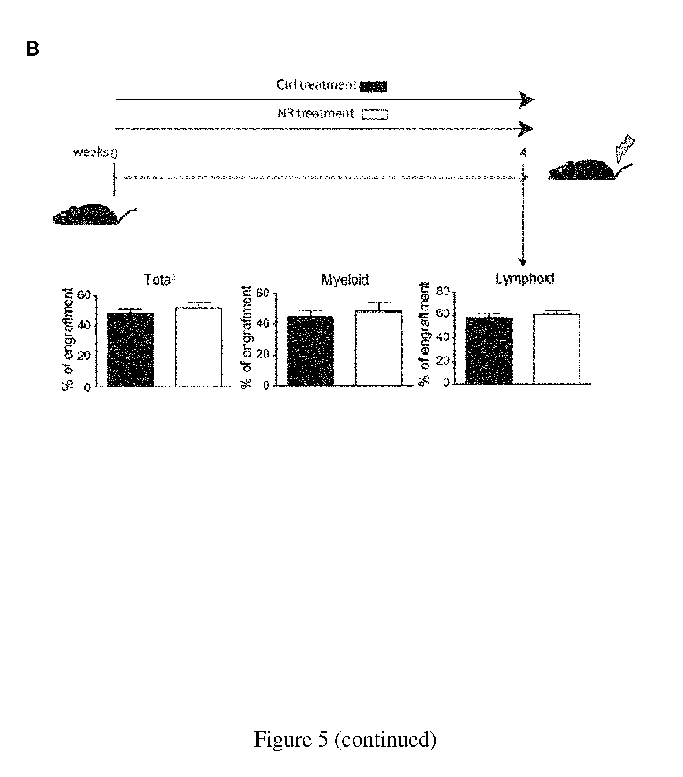

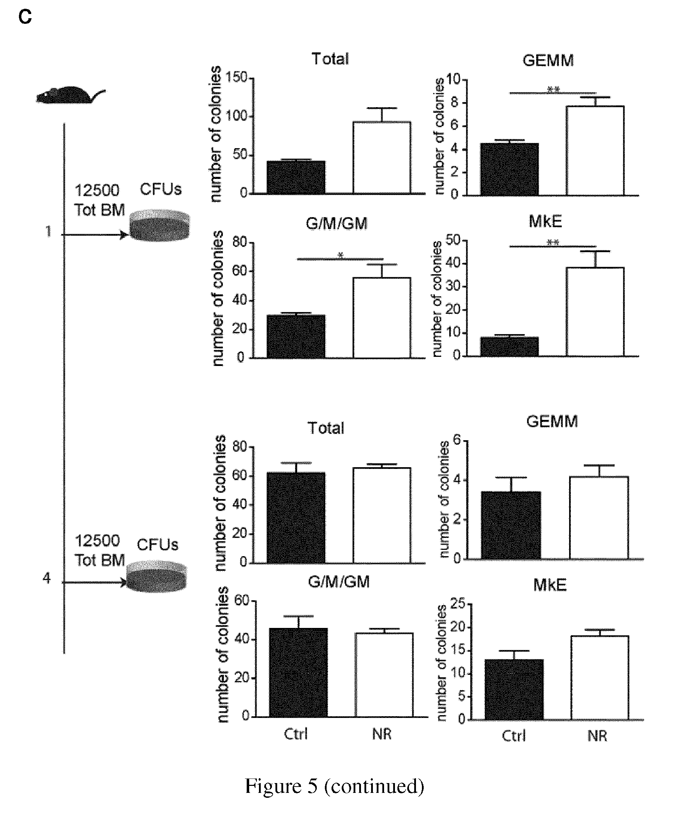

**Figure 5** shows effects of NR treatment in mice by bone marrow analyses on different various cell compartments as described in Example 4. **A:** BM analysis of NR-treated (right) and control (left) mice measured by cell counts by FACS in all hematopoietic progenitor compartments (LT-HSC, ST-HSC, MPP, CP and MEP) after 1 week of treatment (1), after 4 weeks treatment (4). (n=5, statistic t-student); **B:** Long-term 12-week engraftment as measured by congenic CD45.1 percentage chimerism of BM derived from 1-month NR-treated mice (right) in the context of transplantation as compared to control group (left) showing no reduction of HSCs upon NR treatment; **C:** Number of colonies in CFUs assay from BM-derived mice in 1-week-treated group (1) and 4-week-treated group (4) (n=5, statistic t-student).

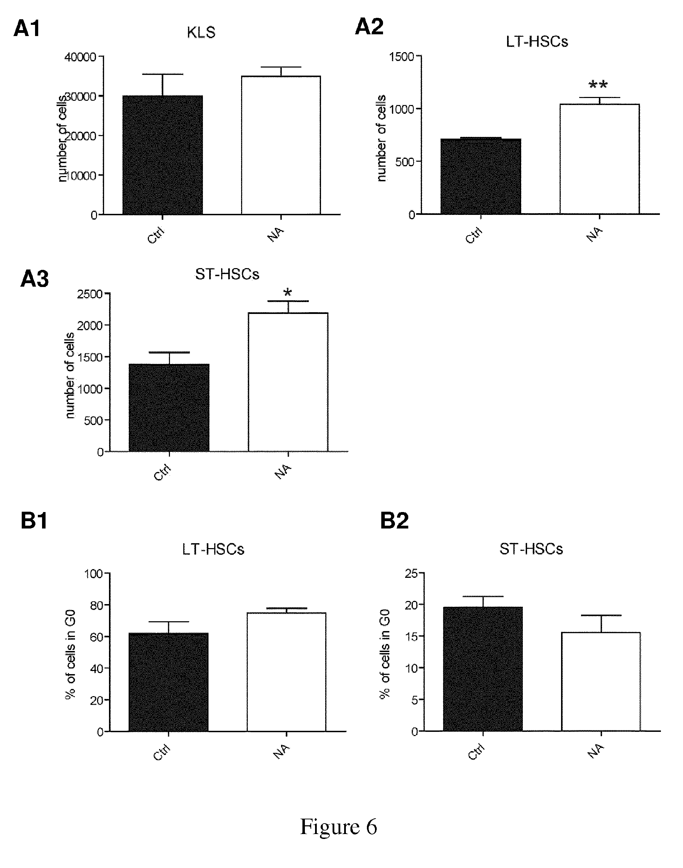

**Figure 6** shows effects of Nicotinic acid (NA) 1-week treatment in mice by bone marrow analyses on different cell compartments as described in Example **5. A1-A3:** FACS analysis of hematopoietic compartments shows an increase of stem and progenitor populations upon NA treatment. **B1-B2**:Cell cycle analysis does not show any significant difference between treated and non-treated conditions.

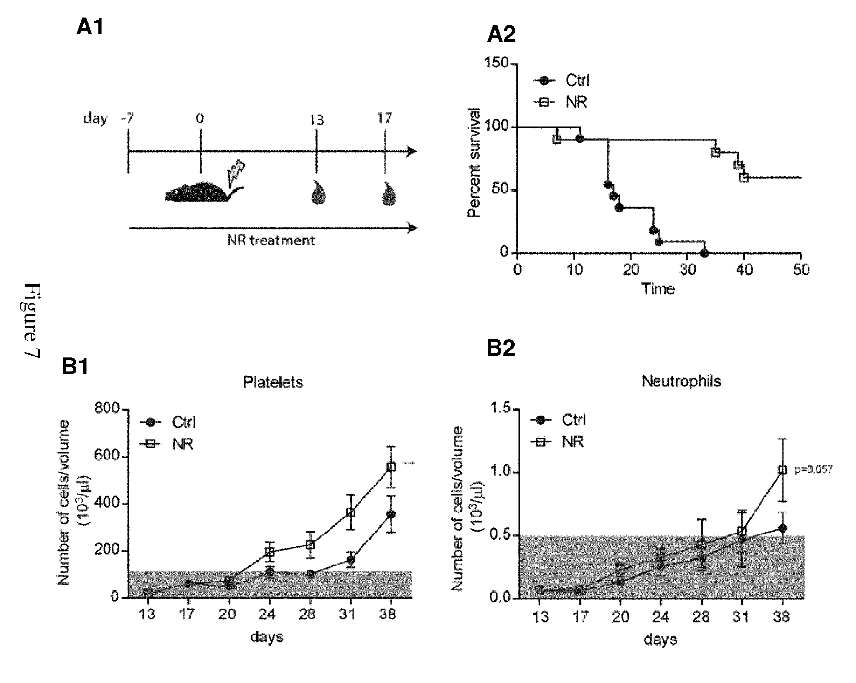

**Figure 7** shows effects of NR treatment in limiting **(A)** and non-limiting **(B)** dose-transplanted mice (square) as compared to controls (dot) as described in Example 5. **A1:** Transplantation protocol; **A2:** Percentage of survival versus time (in days after transplantation) (n=10); **B:** Blood Cell counts versus time (in days after transplantation); **B1:** Platelets (thrombocytopenia zone in grey) and **B2:** Neutrophils (neutropenia zone in grey) (n=10, statistic t-student).

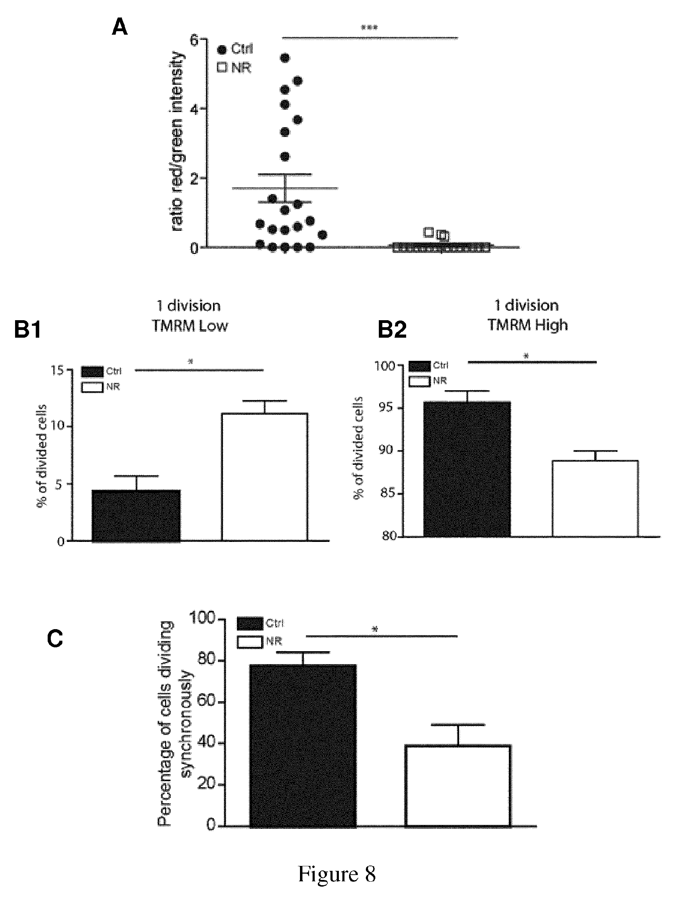

**Figure 8** shows the effects of *in vitro* treatment of HSCs with NR as described in Example 7. **A:** Confocal imaging of JC1 stained HSCs (with TMRM) for mitochondrial in NR-treated and control (CTRL) conditions (n=3, statistic t-student); **B:** Analysis of HSCs populations, namely with low mitochondrial activity ('TMRM low') and with high mitochondrial activity ("TMRM high") after NR-treatment and under control conditions (n=3, statistic t-student); **C:** Percentage of cells dividing synchronously determined by time-lapse microscopy follow-up after NR-treatment and under control conditions (n=3, statistic t-student). As indicated in the right panel, a division was considered as synchronous when both daughter cells divide within a time-frame of 6 hours (Δ t of 3 hours).

Subjects at risk of developing a decrease in blood cell levels include patients suffering from anaemia, undergoing chemotherapy, bone marrow transplant or radiation therapy. Subjects at risk of developing post-transplantation complications include hematopoietic cell depleted subjects having received an autologous or allogeneic hematopoietic stem or progenitor cell graft from primary or *in vitro* manipulated MPBCs, BM or UC.
As used herein the term hematopoietic stem cell (HSC) sample comprises any *ex-vivo* sample comprising hematopoietic stem cell isolated from a source of said cells (e.g., cord blood, bone marrow, peripheral blood, or fetal tissues such as placental tissues, fetal liver or hemogenic endothelium) or multipotent adult progenitor cells (MAPCs) or partially reprogrammed cells from either hematopoietic tissues or other mesodermal or mesodermal-reprogrammed origins.
As used herein the term "hematopoietic stem cell" or "HSC" as used herein, refers to immature blood cells having the capacity to self-renew and to differentiate into more mature blood cells comprising granulocytes (e.g., promyelocytes, neutrophils, eosinophils, basophils), erythrocytes (e.g., reticulocytes, erythrocytes), thrombocytes (e.g., megakaryoblasts, platelet producing megakaryocytes, platelets), monocytes (e.g., monocytes, macrophages, dendritic cells) and lymphocytes (common lymphoid progenitors, pre-B, pro-B, mature B, pre-T, pro-B, mature T and NKT lymphocytes and NK cells). It is also known in the art that hematopoietic stem cells can include pluripotent stem cells, multipotent stem cells (e.g., a lymphoid stem cell), and/or stem cells committed to specific hematopoietic lineages. The stem cells committed to specific hematopoietic lineages may be of T cell lineage, B cell lineage, dendritic cell lineage, Langerhans cell lineage, erythroid, megakaryocytic, myeloid and/or macrophage cell lineage. HSCs also refer to long term HSC (L T-HSC) and short term HSC (ST-HSC). LT-HSC and ST-HSC are differentiated, for example, based on their cell surface marker expression such as described herein or known by the skilled person.
As used herein the term "HSC containing sample" refers to purified preparation of LT-HSCs or unpurified hematopoietic sample (such as total cord blood or total MPBC apheresis product or total bone marrow sample). Purified preparations of LT-HSCs can be prepared through the use of surface markers for phenotypically defined hematopoietic stem and progenitor populations specific to the concerned mammalian species. For example, human LT-HSCs can be purified by using CD34+ surface marker. The efficacy of a method of cell expansion according to the invention can be measured through the measurement of the amount of self-renewing cells in a HSC sample preparation which can be measured phenotypically by flow cytometry through surface immunostaining (i.e. CD34, CD38, Thy, CD133, c-kit, lineage markers) or functionally through the measurement of colony forming units (CFUs) and percentage chimerism in the context of *in vivo* transplant assays.
As used herein, the term "cell expansion culture medium" refers to any standard cell stem cell culture medium suitable for stem cell expansion such as for example culture media described in the following examples or described in Boitano et al., 2010, Science 329, 1345-8.
According to an embodiment, the cell expansion culture medium comprises standard cocktails of cytokines and growth factors. The cocktails of cytokines and growth factors can be used with or without supporting stromal feeder or mesenchymal cells can comprise, but are not restricted to: SCF, TPO, Flt3-L, FGF-1, IGF1, IGFBP2, IL-3, IL-6, G-CSF, M-CSF, GM-CSF, EPO, oncostatin-M, EGF, PDGF-AB, angiopoietin and angiopoietin-like family including Angl5, prostaglandins and eicosanoids including PGE2, Aryl hydrocarbon (AhR) receptor inhibitors such as StemRegenin1 (SR1) and LGC006 *(*Boitano et al., 2010, Science, 329,5997, 1345-1348*).*
As used herein, the term "mitochondrial membrane potential reducing agent" is an agent which is able to induce a reduction in mitochondrial membrane potential such as measured by mitochondrial-potential sensitive dies such as TMRM or MitoTracker Deep Red staining, or by direct measurement of intracellular respiration rate via the seahorse assay. Those agents comprise agents uncoupling electron transport from ATP generation within the mitochondria. Examples of agents uncoupling electron transport from ATP generation within the mitochondria include an agent selected from 2,4 di-nitrophenol (DNP), Carbonyl cyanide 4-(trifluoromethoxy)phenylhydrazone (FCCP), Carbonyl cyanide m-chloro phenyl hydrazine (CCCP), 2-fluorophenyl){6-[(2-fluorophenyl)amino](1,2,5-oxadiazolo[3,4-e]pyrazin-5-yl)}amine (BAM-15) and 4,5,6,7-Tetrachloro-2-trifluoromethylbenzimidazole (TTFB). Identification of any further agents able to uncouple electron transport from ATP generation within the mitochondria can be identified by standard methods known to the skilled person via commercial ATP determination kits. Further mitochondrial membrane potential reducing agents include agents such as nicotinamide riboside (NR), nicotinic acid (NA), niacin, N-formylkynurenine, quinolinic acid, nicotinamide riboside kinase (NRK) activator, nicotinamide mononucleotide (NMN), acipimox or tryptophan.
As used herein, the term "hematopoietic cell depleted subjects" mean subjects presenting a severe neutropenia and/or severe thrombocytopenia and/or severe anemia, such as for example but not limited to post-transplanted subjects or subjects undergoing ablative chemotherapy for solid tumors, patients suffering toxic, drug-induced or infectious hematopoietic failure (i.e. bencene-derivatives, chloranfemicol, B19 parvovirus, etc.) as well as patients suffering from myelodysplastic syndromes, from severe immunological disorders, or from congenital haematological disorders whether of central (i.e. Fanconi anemia) or peripheral origin (i.e. G6PDH deficiency).
As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it for example based on familial history, overweight status or age; (b) inhibiting the disease, i.e., arresting its development; or relieving the disease, i.e., causing regression of the disease and/or its symptoms or conditions such as improvement or remediation of damage.
In particular, treatment of diseases or disorders associated with an absence of haemopoietic function comprises promoting standard blood profile recovery and/ or preventing, or attenuating the risk of infection in hematopoietic cell-depleted patients. The term "subject" as used herein refers to mammals. For example, mammals contemplated by the present invention include human, primates, domesticated animals such as dogs, cats, cattle, sheep, pigs, horses, laboratory rodents and the like.
The term "efficacy" of a treatment or method according to the invention can be measured based on changes in the course of disease or condition in response to a use or a method according to the invention. For example, the efficacy of a treatment or method according to the invention can be measured through the measurement of decrease of hematopoietic cell depletion profile such as the recovery of normal blood cell count or improvement of blood cell counts towards normal cell counts such as for example the exit of and time for reaching the exit of neutropenia (neutrophils > 0.5 G/l, in humans) and/or the exit of and time for reaching the exit of severe thrombocytopenia (platelet count > 100 G/l, in humans) which can be measured by complete peripheral cell blood counts.
Further, the efficacy of a treatment or method according to the invention can be measured through the improvement of the hematopoietic cell depletion profile such through the measurement of CD34+ HSCs cells in peripheral blood or bone marrow, as well as study of the cellularity in the BM smear or biopsy.

### Methods of hematopoietic stem cell expansion according to the invention

According to an embodiment of the invention, is provided a method for *ex-vivo* hematopoietic stem cell expansion comprising the steps of:
a) providing a HSC-containing sample in a cell expansion culture medium;
b) optionally isolating HSC fraction characterized by a low mitochondrial membrane potential from said HSC sample into an isolated HSC pool;
c) contacting HSC sample provided under a) or HSC pool isolated under b) with at least one mitochondrial membrane potential reducing agent or a mixture thereof.

According to a further embodiment, is provided a method for *ex-vivo* hematopoietic stem cell expansion according to the invention wherein the HSC sample is a bone marrow sample and/or bone marrow cell preparation.
According to a further embodiment, is provided a method for *ex-vivo* hematopoietic stem cell expansion according to the invention wherein the HSC sample is a UCB sample or UCB cell preparation.
According to a further embodiment, is provided a method for *ex-vivo* hematopoietic stem cell expansion according to the invention wherein the HSC fraction with low mitochondrial membrane potential is isolated by using fluorescence dyes selected from JC1, TMRM and MitoTracker DeepRed..
According to a further embodiment, is provided a method for *ex-vivo* hematopoietic stem cell expansion according to the invention wherein the said at least one mitochondrial membrane potential reducing agent is an agent uncoupling electron transport from ATP generation within the mitochondria.
According to another further embodiment, is provided a method for *ex-vivo* hematopoietic stem cell expansion according to the invention wherein the said at least one mitochondrial membrane potential reducing agent is selected from the following group: 2,4 di-nitrophenol (DNP), Carbonyl cyanide 4-(trifluoromethoxy)phenylhydrazone (FCCP), Carbonyl cyanide m-chloro phenyl hydrazine (CCCP), 2-fluorophenyl){6-[(2-fluorophenyl)amino](1,2,5-oxadiazolo[3,4-e] pyrazin-5-yl)}amine (BAM-15) and 4,5,6,7-Tetrachloro-2-trifluoromethyl benzimidazole (TTFB).
According to another further embodiment, is provided a method for *ex-vivo* hematopoietic stem cell expansion according to the invention wherein the said at least one mitochondrial membrane potential reducing agent is carbonyl cyanide 4-(trifluoromethoxy)phenylhydrazone.
According to a further embodiment, is provided a method for *ex-vivo* hematopoietic stem cell expansion according to the invention wherein the said at least one mitochondrial membrane potential reducing agent is an agent selected from nicotinamide riboside (NR), nicotinic acid (NA), niacin, N-formylkynurenine, quinolinic acid, nicotinamide riboside kinase (NRK) activator, nicotinamide mononucleotide (NMN), acipimox or tryptophan.
According to another further embodiment, is provided a method for *ex-vivo* hematopoietic stem cell expansion according to the invention wherein the said at least one mitochondrial membrane potential reducing agent is nicotinamide riboside (NR). According to another further embodiment, is provided a method for *ex-vivo* hematopoietic stem cell expansion according to the invention wherein the said at least one mitochondrial membrane potential reducing agent is nicotinic acid (NA). According to a further embodiment, is provided a method for *ex-vivo* hematopoietic stem cell expansion, wherein the cell preparation obtained after step b) or c) is enriched in functional stem cells having long-term multi-lineage blood reconstitution capability. According to a further embodiment, is provided a method for *ex-vivo* hematopoietic stem cell expansion, wherein stemness (e.g. self-renewing capacity of human HSCs is assessed by quantifying CD34+ cells and by transplantation assays) of the cell preparation obtained after step c) is maintained for longer time after sample taking and/or increased as compared to a HSC sample in absence of a mitochondrial membrane potential reducing agent.
According to a further embodiment, is provided a method for cryoprotecting either HSCs or short-term progenitors, the later ones being very sensitive to freezing. Uncoupling agents will be used at concentration between 1-50 µM.
According to another embodiment, is provided a cell expansion culture medium comprising at least one mitochondrial membrane potential reducing agent.
According to another embodiment, is provided a cell expansion culture medium comprising at least one mitochondrial membrane potential reducing agent and further comprising a cocktail of cytokines and growth factors useful for stem cell expansion with optionally supporting stromal feeder or mesenchymal cells such as SCF, TPO, Flt3-L, FGF-1, IGF1, IGFBP2, IL-3, IL-6, G-CSF, M-CSF, GM-CSF, EPO, oncostatin-M, EGF, PDGF-AB, angiopoietin and angiopoietin-like family including Angl5, prostaglandins and eicosanoids including PGE2, Aryl hydrocarbon receptor inhibitors such as StemRegenin1 (SR1) and LGC006.
According to another embodiment of the invention, is provided a kit for selecting functional stem cells having long-term multi-lineage blood reconstitution capability, said kit comprising i) at least one detecting agent for surface marker for phenotypically defined hematopoietic stem and progenitor cell or a combination of several of those markers and ii) at least one agent for detecting low mitochondrial membrane potential in hematopoietic stem and progenitor cells, such as JC1, TMRM or a Mitotracker® dye (Benzoxazolium, 2-[3-[5,6-dichloro-1,3-bis[[4-(chloromethyl)phenyl]methyl]-1,3-dihydro-2H-benzimidazol-2-ylidene]-1-propenyl]-3-methyl-, chloride/ 201860-17-5). According to a further embodiment of the invention, is provided a kit according to the invention, wherein the said at least one detecting agent for phenotypically defined hematopoietic stem and progenitor cell is one or more selective antibody directed to said surface marker selected from c-Kit, Lin-, Sca-1, CD150, CD34+ and CD48-. According to a further embodiment of the invention, is provided a kit or a cell expansion culture medium for hematopoietic stem cell comprising at least one mitochondrial membrane potential reducing agent selected from an agent uncoupling electron transport from ATP generation within the mitochondria and an agent selected from nicotinamide riboside (NR), nicotinic acid (NA), niacin, N-formylkynurenine, quinolinic acid, nicotinamide riboside kinase (NRK) activator, nicotinamide mononucleotide (NMN), acipimox or tryptophan.
According to a further embodiment, is provided a kit or a cell expansion culture medium for hematopoietic stem cell further comprising at least one agent uncoupling electron transport from ATP generation within the mitochondria.
According to a further embodiment, is provided a kit or a cell expansion culture medium for hematopoietic stem cell according to the invention, wherein said at least one agent uncoupling electron transport from ATP generation within the mitochondria is selected from the following group: 2,4 di-nitrophenol (DNP), Carbonyl cyanide 4-(trifluoromethoxy)phenylhydrazone (FCCP), Carbonyl cyanide m-chloro phenyl hydrazine (CCCP), 2-fluorophenyl){6-[(2-fluorophenyl)amino](1,2,5-oxadiazolo[3,4-e] pyrazin-5-yl)}amine (BAM-15) and 4,5,6,7-Tetrachloro-2-trifluoromethyl benzimidazole (TTFB).
According to another aspect the invention provides a method for identifying a candidate for the maintenance and/or increase of stemness of cells in a HSC sample comprising:
(a) contacting a HSC sample with a candidate substance to be tested;
(b) optionally isolating HSC fraction characterized by a low mitochondrial membrane potential from said HSC sample into an isolated HSC pool;
(c) measuring whether said substance to be tested lowers the mitochondrial membrane potential of said cells in said HSC sample under step a) or isolated HSC pool isolated under step b) and/or increases the population of cells having a low mitochondrial membrane potential in said HSC sample under step a) or isolated HSC pool isolated under step b); and
(d) identifying said substance.

According to a further aspect, the invention provides method for identifying a candidate for the maintenance and/or increase of stemness of cells in a HSC sample, wherein mitochondrial membrane potential in said cells in measured under step c) by using a fluorescence dye such as JC1 or TMRM.
The method of cell expansion according to the invention or stem cell preparations obtained by the method according to the invention or by using the kit of preparation of the invention can be further used in known methods for hematopoietic stem cell expansion such as described in *Boitano et ak.2010, supra* and for use in transplantation protocols according to known methods such as described in Boitano et *al., 2010, supra.* The population of cells obtained by method of cell expansion according to the invention can be formulated for clinical haemopoietic stem cell transplantation, or for augmentation of haemopoietic function in a subject in need thereof.

### Use according to the invention

According to an embodiment, the invention provides a mitochondrial membrane potential reducing agent for use in the prevention and/or treatment of a disease or disorder associated with a decreased blood cell level, for example resulting from a decreased or an absence of a haemopoietic function such as severe neutropenia, anemia and/or thrombocytopenia, in particular in hematopoietic stem cell post-transplanted subjects or subjects undergoing ablative chemotherapy for solid tumors or suffering from severe immunological disorders.
According to another embodiment of the invention provides a use of a mitochondrial membrane potential reducing agent for the preparation of a medicament or a food supplement for the prevention and/or treatment of a disease or disorder associated with a decreased blood cell level, for example resulting from a decreased or an absence of a haemopoietic function such as severe neutropenia and/or thrombocytopenia, in particular in hematopoietic stem cell post-transplanted subjects or subjects undergoing ablative chemotherapy for solid tumors or suffering from severe immunological disorders.
According to an embodiment, the invention provides a method of preventing and/or treating a decreased blood cell level as compared to a control blood cell level in a subject, said method comprising administering an effective amount of a mitochondrial membrane potential reducing agent in a subject by injection or as a food supplement. According to an embodiment, the invention provides a method for promoting standard blood profile recovery, or attenuating the risk of infection in hematopoietic cell-depleted patients, said method comprising administering an effective amount of a membrane potential reducing agentin a subject by injection or as a food supplement. According to another embodiment, the membrane potential reducing agent according to the invention is able to increase the proportion of cells with low mitochondrial potential which can be measured by known techniques and techniques as described herein. According to a further embodiment, the membrane potential reducing agent is selected from nicotinamide riboside (NR), niacin, nicotinic acid (NA), N-formylkynurenine, quinolinic acid, nictotinamide riboside kinase (NRK) activator, nicotinamide mononucleotide (NMN), acipimox and tryptophan.
According to a further embodiment, the membrane potential reducing agent is nicotinamide riboside.
According to a further embodiment, the membrane potential reducing agent is nicotinic acid.
According to a further embodiment, the membrane potential reducing agent is useful in inducing blood recovery by faster exit of neutropenia and/or thrombocytopenia via increased neutrophil and platelet recovery.
The method of cell expansion according to the invention or stem cell preparations obtained by the method according to the invention or by using the kit of preparation of the invention or methods of treatment according to the invention would also provide benefits in forming a supplemental treatment to chemotherapy, or provide assistance in the treatment of other disease states concerning an alteration of hematopoietic cells, and may also provide a further application for long-term stem cell cultures.

### Compositions according to the invention

The invention provides mitochondrial membrane potential reducing agents useful for use in the prevention and/or treatment of of a disease or disorder associated with an absence of haemopoietic function such as severe neutropenia and/or thrombocytopenia. Membrane potential reducing agent or formulations thereof may be administered as a pharmaceutical formulation or a food supplement, which can contain one or more agents according to the invention in any form described herein. The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.
Compositions of this invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.
Compositions of this invention may also be formulated as a depot preparation, which may be administered by implantation or by intramuscular injection.
Solid compositions of this invention may be in the form of tablets or lozenges formulated in a conventional manner. For example, tablets and capsules for oral administration may contain conventional excipients including, but not limited to, binding agents, fillers, lubricants, disintegrants and wetting agents. Binding agents include, but are not limited to, syrup, accacia, gelatin, sorbitol, tragacanth, mucilage of starch and polyvinylpyrrolidone. Fillers include, but are not limited to, lactose, sugar, microcrystalline cellulose, maize starch, calcium phosphate, and sorbitol. Lubricants include, but are not limited to, magnesium stearate, stearic acid, talc, polyethylene glycol, and silica. Disintegrants include, but are not limited to, potato starch and sodium starch glycollate. Wetting agents include, but are not limited to, sodium lauryl sulfate.
Tablets may be coated according to methods well known in the art.
The compounds of this invention can also be administered in sustained release forms or from sustained release drug delivery systems.
For post-transplant or post-chemotherapy patients parenteral administration will be preferred, as post-chemotherapy mucitis often impairs all oral intake, some patients even requiring parenteral nutrition. According to a particular embodiment, compositions according to the invention are for intravenous use.
According to a particular embodiment, compositions according to the invention might be administered to a subjected as food supplement.
According to one aspect, the food supplement formulation may be taken orally at a dosage rate in membrane potential reducing agent ranging from about 10 to about 50 mg/kg/day. Typically, the dosage rate of nicotinic acid according to the invention can be in slow release form at a dosage rate ranging from about 10 to about 50 mg/kg/day (e.g acipinox).
In another particular aspect, the compositions according to the invention are adapted for delivery by repeated administration.
According to a particular embodiment, compositions of the invention are veterinary compositions.

Further materials as well as formulation processing techniques and the like are set out in *Part 5 of* Remington's "The Science and Practice of Pharmacy", 22nd Edition, 2012, University of the Sciences in Philadelphia, Lippincott Williams & Wilkins*,* which is incorporated herein by reference.

### Mode of administration

Mitochondrial membrane potential reducing agentss or formulations thereof may be administered in any manner including orally, parenterally, intravenously, rectally, or combinations thereof Parenteral administration includes, but is not limited to, intravenous, intra-arterial, intra-peritoneal, subcutaneous and intramuscular. The compositions of this invention may also be administered in the form of an implant, which allows slow release of the compositions as well as a slow controlled i.v. infusion. According to a particular aspect, mitochondrial membrane potential reducing agents or formulations thereof are administered as food supplement.
According to a particular aspect, mitochondrial membrane potential reducing agents or formulations thereof are administered as injectable formulations.
The dosage administered, as single or multiple doses, to an individual will vary depending upon a variety of factors, including pharmacokinetic properties, patient conditions and characteristics (sex, age, body weight, health, size), extent of symptoms, concurrent treatments, frequency of treatment and the effect desired.

### Combination

The invention having been described, the following examples are presented by way of illustration, and not limitation.
According to the invention, mitochondrial membrane potential reducing agents or formulations thereof, including pharmaceutical formulations thereof can be administered alone or in combination with a co-agent e.g. multiple drug regimens) useful for preventing or treating a disease or disorder associated with an absence of haemopoietic function, or useful for promoting blood recovery and/ or preventing, or attenuating the risk of infection in hematopoietic cell-depleted patients.
The invention encompasses the administration of a compound of the invention or a formulation thereof according to the invention wherein it is administered to a subject prior to, simultaneously or sequentially with other therapeutic regimens or co-agents useful for preventing or treating a disease or disorder associated with an absence of haemopoietic function, or useful for promoting blood recovery and/ or preventing, or attenuating the risk of infection in hematopoietic cell-depleted patients.

A compound of the invention or a formulation thereof according to the invention that is administered simultaneously with said co-agents can be administered in the same or different composition(s) and by the same or different route(s) of administration. According to one embodiment, is provided a pharmaceutical formulation comprising a mitochondrial membrane potential reducing agents, combined with at least one co-agent useful for preventing or treating a disease or disorder associated with an absence of haemopoietic function, or useful for promoting blood recovery and/ or preventing, or attenuating the risk of infection in hematopoietic cell-depleted patients such as G-CSF analogues (i.e. filgrastim) which is used for the treatment of neutropenia or TPO receptor analogues (i.e. N-PLATE) which are used for the treatment of autoimmune thrombocytopenia.

### Patients

In an embodiment, patients according to the invention are subjects suffering from disease or disorders associated with a decreased blood cell level resulting from a reduction or an absence of haemopoietic function or patients at risk of developing a decreased blood cell level as compared to a control blood cell level.
In another embodiment, patients according to the invention are hematopoietic cell depleted subjects.
In a further embodiment, the decreased blood cell level is secondary to a primary or autoimmune disorder of the hematopoietic system, for example including, but not limited, to congenital bone marrow failure syndromes, idiopathic thrombocytopenia, aplastic anemia and myelodysplastic syndromes.
In an embodiment, patients according to the invention are subjects suffering from severe neutropenia and/or thrombocytopenia or suffering from severe immunological disorders.
In a particular embodiment, subjects according to the invention are hematopoietic stem cell post-transplanted subjects.
In a particular embodiment, subjects according to the invention are suffering from haematological and immunological disorders and cancers.

In a particular embodiment, subjects according to the invention are subjects undergoing ablative chemotherapy for a neoplasm.
In a particular embodiment, subjects according to the invention are suffering from a haematological cancer.
In a further particular embodiment, subjects according to the invention are suffering from a haematological cancer selected from leukemia and lymphoma.
In another further particular embodiment, patients according to the invention are subjects with decreased blood cell level as a pharmacological side-effect.
In another further particular embodiment, subjects according to the invention are subjects that have limited bone marrow reserve such as elderly subjects or subjects previously exposed to an immune depleting treatment such as chemotherapy.
In another further particular embodiment, subjects according to the invention have a decreased blood cell level or are at risk for developing a decreased blood cell level as compared to a control blood cell level. Those subjects include subject suffering from blood cancers (e.g., leukemia, lymphoma), blood disorders (e.g., inherited anemia, inborn errors of metabolism, aplastic anemia, beta-thalassemia, Blackfan-Diamond syndrome, globoid cell leukodystrophy, sickle cell anemia, severe combined immunodeficiency, X-linked lymphoproliferative syndrome, Wiskott-Aldrich syndrome, Hunter's syndrome, Hurler's syndrome Lesch Nyhan syndrome, osteopetrosis), subjects undergoing a chemotherapy rescue of the immune system, and other diseases (e.g., autoimmune diseases, diabetes, rheumatoid arthritis, system lupus erythromatosis). Furthermore, subjects according to the invention include subjects presenting a severe neutropenia and/or severe thrombocytopenia and/or severe anemia, such as for example but not limited to post-transplanted subjects or subjects undergoing ablative chemotherapy for solid tumors, patients suffering toxic, drug-induced or infectious hematopoietic failure (i.e. bencene-derivatives, chloranfemicol, B19 parvovirus, etc.) as well as patients suffering from myelodysplastic syndromes, from severe immunological disorders, or from congenital haematological disorders whether of central (i.e. Fanconi anemia) or peripheral origin (i.e. G6PDH deficiency).
References cited herein are hereby incorporated by reference in their entirety. The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

### EXAMPLES

**Sca1** (Stem cell antigen 1), **SCF** (Stem cell factor), **TPO** (Thrombopoietin), **FGF-1** (fibroblast growth factor 1), **IGFBP2** (Insulin growth factor binding protein 2).

### Example 1: Identification of mitochondrial membrane potential signature of HSC sub-populations with long-term reconstitution capacity

Mitochondrial membrane potential (ΔΨm), as indicated by tetramethylrhodamine methyl ester (TMRM) fluorescence was used to follow the metabolic state of HSC and MPP *in vitro* and *in vivo* as follow.

### Phenotypically defined HSC and progenitor cells show distinct ΔΨm in vitro

HSCs and their closely related offspring were separated by the most commonly used combinations of surface markers for phenotypically defined hematopoietic stem and progenitor populations (Committed progenitors, CPs: c-Kit+; LKS: Lin- c-Kit+ Sca-1+ (i.e. a population that comprises all multipotent stem and progenitor cells in the bone marrow); short-term HSCs: LKS CD150+ CD34+ (ST-HSC); long-term HSCs: LKS CD150+ CD34- (LT-HSC)) by using the corresponding antibodies defined below. ΔΨm levels were analyzed by flow cytometry and confocal microscopy on those phenotypically cells labeled with TMRM a cell-permeable dye that is readily sequestered by active mitochondria, in order to report mitochondrial polarization that correlates with the level of OXPHOS in a cell *(*Folmes et al., 2011, Cell Metab 14, 264-271), following the protocols defined below.

### Flow cytometry and fluorescence activated cell sorting (FACS)

Bone marrow (BM) cells were isolated from C57B1/6J and C57B1/6J Ly5.1 from crushed femora and tibia. Flow cytometry analysis of hematopoietic stem and progenitor cells was performed on freshly isolated bone marrow (BM). Cell suspensions were filtered through a 70 µm cell strainer and erythroid cells were eliminated by incubation with red blood cells lysis buffer (eBioscences). Lineage-positive cells were removed with a magnetic lineage depletion kit (Miltenyi Biotech). Cell suspensions were stained with a panel of specific antibodies for stem and progenitor cells and analyzed or FACS-sorted respectively on a BD LSRII and BD FACS Aria II.

### Analysis of mitochondrial activity

Freshly isolated BM cells were incubated at 37°C for one hour with 200 nM TMRM (Invitrogen) and then stained with following specific antibodies for the different hematopoietic stem/progenitor cell compartments: rat mAbs against cKit (2B8), Sca1 (D7), CD150 (TC-15-12F12.2), CD34 (RAM34), CD45.2 (104), CD45.1 (A20), Gr1 (RB6-8C5), F4/80 (BM8), CD19 (6D5), CD3 (17A2), CD16/CD32 (2.4G2) which were purchased from Biolegend, eBiosciences and BD (Becton, Dickinson and Company). A mixture of biotinylated mAbs against CD3, CD11b, CD45R/B220, Ly-6G, Ly-6C and TER-119 was used as lineage cocktail (BD). Labeled cells were FACS-sorted or analyzed by flow cytometry.
For confocal imaging, LT-HSC, ST-HSC, MPPs and committed progenitors were sorted and placed on adherent poly-L-lysine (PLL)-coated glass slides for six hours. 20 nM TMRM or JC1 (Cayman Chemical) was then added in the media and live cell images were acquired on a Leica SP5 confocal microscope. For MitoTracker® Deep Red (Invitrogen) staining, cells were incubated at 200 nM for one hour at 37°C. NADH autofluorescence was measured by flow cytometry with UV laser (ex: 350 nm, em: 460 nm).
Each population displayed a distinct level of TMRM intensity, with a stepwise increase from the most primitive to the most committed population as shown by flow cytometry **(****Fig. 1** **A**) and microscopy-based read-outs **(****Fig. 1B**)**:** ΔΨm was lowest in the LT-HSCs population **(****Fig. 1A**), becoming barely detectable by confocal microscopy **(****Fig. 1** **B**).
These data support that mitochondrial metabolism is limited in the most primitive quiescent HSC population, most likely as a strategy to protect these rare and long-lived cells from cellular damage inflicted by ROS production. In marked contrast, hematopoietic progenitors that are highly proliferative meet their increasing energy requirement by increasing mitochondrial metabolism.

### Low ΔΨm exclusively marks HSC subpopulations with long-term blood reconstitution capacity in vivo

Using *in vivo* multi-lineage blood reconstitution assays, it was tested in phenotypically defined LKS (a population that contains all multipotent stem and progenitor cells in the bone marrow), ST- and LT-HSCs to which extent ΔΨm levels could report long-term stem cell function.
To this end, LKS subpopulations were isolated by FACS and separated by low (LKS:TMRM^{low}) and high (LKS:TMRM^{high}) ΔΨm levels. Read out ranges for LKS:TMRM^{low} are ∼ 30% low population and for LKS:TMRM^{high} are ∼ 30% high population. Transplantation of these two phenotypes into lethally irradiated mice was performed using a double congenic allelic system as described below.

### Transplantations

C57B1/6 Ly5.2 mice were lethally irradiated at 850RAD and transplanted with donor cells derived from C57B1/6 Ly5.1 mice and competitor cells derived from C57B1/6 Ly5.1/5.2 mice. For LKS transplants, 1000 LKS (TMRM^{low} or TMRM^{high}) donor cells were transplanted together with 250*10³ total BM competitor cells in recipient mice. For LKS CD150+CD34- ('LT-HSC') and LKS CD150+ CD34+ ('ST-HSC') transplants, 80 LT-HSC (TMRM^{low} or TMRM^{high}) or 80 ST-HSC (TMRM^{low} or TMRM^{high}) were transplanted together with 250*10³ total BM competitor cells in recipient mice.
Peripheral blood was collected at 4, 8 and 16 weeks to determine the repopulating units (RU) of donor-derived cells and their differentiation state by flow cytometry analysis. One RU is equivalent to the repopulating ability found in 100,000 competitor bone marrow cells.
For transplantation of *in vitro*-cultured LT-HSCs, the progeny of 200 LT-HSCs cultured for five days were FACS-sorted based on their TMRM signal (TMRM^{low} or TMRM^{high}) and transplanted together with 2*10⁶ helper cells. Helper cells were derived from BM of C57B1/6 Ly5.1/5.2 mice that were depleted for Sca1 and CD150 positive cells (Miltenyi Biotech). Peripheral blood was collected at 4, 8 and 16 weeks to determine the percentage of chimerism. Hematopoietic chimerism is a measure of the number of donor and recipient cells in the host following stem cell transplantation (SCT) and was measured by peripheral blood cells analysis.
For the CFSE-TMRM transplants, LT-HSCs were sorted and stained for CFSE as described below. At the end of a 2-day culture period, cell progeny having undergone one division were stained with TMRM and re-sorted based on TMRM^{low} and TMRM^{high} signals. Each recipient mouse was injected with 100 cells of either population together with 2*10⁶ helper cells. Peripheral blood was collected at 4, 8 and 16 weeks to determine the percentage of chimerism.
Secondary Transplantation: Secondary recipients C57B1/6 Ly5.2 mice were lethally irradiated at 850RAD and transplanted with 3 million bone marrow cells from the primary recipients. Peripheral blood was collected at 4 and 8 weeks to determine the level of chimerism.
Long-term multi-lineage blood reconstitution analysis showed that within LKS, only cells with low ΔΨm (*i.e.* LKS:TMRM^{low}) show long-term multi-lineage reconstitution **(****Fig. 2A**). Therefore, the addition of a metabolic marker (low ΔΨm) to the existing surface marker repertoire allows purification of cells with long-term reconstitution capacity from a poorly defined population (LKS) containing mainly MPPs.
Using the same sorting strategy, ST-HSCs, ST-HSC:TMRM^{low} and ST-HSC:TMRM^{high} were compared for their ability to reconstitute the blood system **(****Fig. 2 B)** and strikingly, within the ST-HSC population, short-term multi lineage reconstitution capacity was almost exclusively restricted in the TMRM^{low} fraction **(****Fig. 2B****).** Moreover, phenotypically defined LT-HSCs could be separated into two functionally very distinct populations and only the population with low mitochondrial activity (LT-HSC:TMRM^{low}, corresponding to ∼55% of the population) was capable of long-term multi-lineage reconstitution, whereas LT-HSC:TMRM^{high} cells completely failed to do so **(****Fig. 2C****).** Importantly, propidium iodide staining did not show any difference in viability between the four populations analyzed, ruling out the possibility that lack of engraftment was caused by differential cell death.
Those *in vivo* data reveal a striking functional heterogeneity in phenotypically defined HSCs. The observation that LT-HSCs with activated mitochondria *(i.e.* LT-HSC:TMRM^{high}) do not even reconstitute blood in the short-term **(****Fig. 2C****),** suggests that these cells may not be hierarchically related to 'true' LT-HSCs. They may instead represent HSCs that give rise to long-term lineage-restricted progenitor cells, as shown by recent *in vivo* single-cell multi-lineage reconstitution assays performed on the same immunophenotypes *(*Yamamoto et al., 2013, Cell 154, 1112-1126*).* To test whether HSC TMRM^{high} cells versus TMRM^{high} cells within the LT-HSC compartment contain different levels of such lineage-biased cell types, CD41 was used a functional marker for megakaryocytes, to further separate the two phenotypes. Previous studies have shown that CD41-expressing cells in the HSC compartment have a strong myeloid bias *(*Gekas et al., 2013, Blood 121, 4463-4472*)* and only short- or intermediate-term repopulation activity *(Yamamoto et al., 2013, supra).* In the present experiment, LT-HSC:TMRM^{low} cells comprise approximately 70% of cells that are negative for CD41, in marked contrast to LT-HSC:TMRM^{high} cells where the majority of cells is CD41 positive.
Therefore, the observed functional heterogeneity can be at least partially explained by the presence of CD41+ megakaryocyte progenitors that might have different ΔΨm levels compared to functional LT-HSCs.

### Transcriptome analysis links loss of stemness to an induction of mitochondrial path ways

Next, the four newly identified HSC phenotypes were studied to identify genes and pathways differentially expressed among them using Affymetrix Mouse Gene 1.0 ST arrays as described below. Gene ontology analysis showed that the most modulated genes correspond to cell cycle and mitochondrial pathways. Both pathways are differentially regulated in strikingly opposite directions between LT-HSC:TMRM^{low} and ST-HSC:TMRM^{high} cells. These pathways are down-regulated in the most stem-like population while they increase in the least primitive HSC compartments. The two intermediate populations (LT-HSC:TMRM^{high} and ST-HSC:TMRM^{low}) show roughly equal activity of up- and down-regulation of cell cycling and mitochondrial activity genes. Notably, LT-HSC:TMRM^{low} cells, corresponding to the population capable of long-term multilineage reconstitution *in vivo,* are characterized by very low expression levels of citric acid (or tricarboxylic acid, TCA) cycle genes, in marked contrast to all other populations. The TCA cycle in mitochondria is closely linked to OXPHOS. Therefore, these data suggest that the most self-renewing HSCs have mechanisms in place to attenuate the TCA cycle, presumably to protect them from cellular damage inflicted by ROS.

### Quiescent and cycling HSCs in the bone marrow have similar ΔΨm profiles

Based on the correlation between low ΔΨm and *in vivo* function shown above **(****Fig. 2****),** in the native bone marrow, quiescent and cycling LT-HSC might have similar levels of mitochondrial activity, *i.e.* anaerobic glycolysis would be a hallmark of stemness independent of the cell cycle state.
In order to verify this hypothesis, cell cycle phase analyses were performed using Ki67 and Hoechst staining as described below on freshly isolated HSCs that were separated into low and high ΔΨm. Since cell cycle staining involves cell fixation that is not compatible with TMRM-based assays, MitoTracker® Deep Red was used as a marker for ΔΨm (Simsek et al., 2010, Cell Stem Cell 7, 380-390*)* which was controlled for labeling ΔΨm in stem and progenitor cells in a comparable manner to TMRM. Under homeostatic conditions, quiescent state, corresponding to ca. 70% of cells, and cycling HSCs have indistinguishable ΔΨm profiles.
A similar analysis was performed on HSCs that were activated to exit dormancy directly *in vivo* using interferon-alpha (IFN-α) treatment of mice *(*Essers et al., 2009, Nature 458, 904-908*)* through subcutaneous injections of IFN-α carried out 48 and 24 hours prior to cell isolation. Compared to control mice, IFN-α treatment resulted in a significant increase in the number of cycling (Ki67⁺) HSCs from ca. 30 to 50% in line with previous data (*Essers et al., 2009, supra).* However, just like under homeostatic conditions, ΔΨm levels of quiescent and cycling HSCs were not significantly different.

These data show that *in vivo,* a low ΔΨm is not a unique hallmark of the quiescent LT-HSC state but seems to be indicative of functional HSCs independent of their cell cycle status.

### Cell cycle staining

Sorted HSCs were fixed and permeabilized using Cytofix/Cytoperm plus kit (BD), according to the manufacturer instruction. Fixed cells were then stained overnight with FITC Ki67 (BD) at 4°C, and 10 minutes by Hoechst33342 (Invitrogen).

### In vivo activation of HSC

HSC were activated to exit dormancy by interferon-alpha (IFN-α) treatment following published protocols (*Essers et al., 2009, supra*)*.* Briefly, subcutaneous injections in C57B1/6J mice were carried out with 10,000U of IFN-α (R&D systems) 48 and 24 hours prior to bone marrow extraction. Control mice were injected with an equivalent volume of the vehicle (PBS+0.1%BSA).

### Example 2: Low mitochondrial membrane potential as a hallmark of self-renewal divisions of HSCs in vitro

Having established low ΔΨm as a functional discriminator of stemness **(****Fig. 2B,C****)** and revealing comparable ΔΨ m levels of quiescent and cycling LT-HSCs *in vivo,* it was studied whether ΔΨ m levels would discriminate self-renewing from differentiating HSCs in heterogeneous bulk cultures **(****Fig. 3****).** When HSCs are cultured *in vitro,* the combinatorial expression of surface markers fails to predict their *in vivo* function when re-grafted into irradiated mice *(*Zhang et al., 2005, Blood 105, 4314-4320*).* Indeed, there is currently no strategy that allows the distinction of LT-HSC from their earliest progeny, which already have a restricted multi-lineage blood reconstitution capacity. To test whether TMRM-based read-outs could be used to tell apart self-renewal from differentiation divisions, 200 LT-HSCs from CD45.1 donor mice were isolated based on surface marker expression (Lin-, Sca1+, cKit+, CD150+ and CD34-) and expanded in *vitro* as described below in serum-free medium containing a cocktail of the self-renewing factors Angiopoietin-like protein, Insulin-like growth factor binding protein 2, stem cell factor, fibroblast growth factor 1 and thrombopoietin as described by Huynh et al., 2008, Stem Cells 26, 1628-1635 **(****Fig. 3A**). After 5 days of cell expansion, the cells were resorted by FACS based on TMRM^{low} and TMRM^{high} phenotypes, and transplanted into lethally irradiated CD45.2 recipient mice as described above. Consistent with presults using low TMRM fluorescence to directly isolate functional HSCs *in vivo* as presented in Example 1 above, a low TMRM signal was also predictive of the long-term blood reconstitution capacity of cultured HSCs **(****Fig. 3B****).** Analysis of peripheral blood chimerism by FACS analysis, four months after grafting the cultured HSCs showed that TMRM^{low} cells induce significantly higher long-term multi-lineage blood reconstitution levels compared to cells that had a high TMRM signal (TMRM^{high} cells). This suggested that self-renewing HSCs in culture can be detected based on the same metabolic hallmark as freshly isolated HSCs from the bone marrow **(****Fig. 2****).** However, since it is known that a small fraction of cultured HSCs can maintain their stem cell potential by remaining quiescent even after prolonged time in culture (e.g. Lutolf et la., 2009, Integer Biol (Camb) 1, 59-69*),* the reconstitution from TMRM^{low} cells seen in **Fig. 3B** might have come from non-dividing HSCs. To exclude this possibility and unequivocally identify self-renewing cells in these bulk cultures in order to track the precise number of cell divisions a mother HSC had undergone, LT-HSCs were uniformly labeled with carboxyfluorescein succinimidyl ester (CFSE) as described below, a live cell-permeable dye that is diluted with every cell division such that CFSE intensity is decreased by ∼50% upon each division. By these means, cultured HSC daughter cells that underwent precisely one division after two days in expansion culture were FACS-sorted and further separated them into TMRM^{low} and TMRM^{high} sub-populations. Hundred (100) cells of each population were transplanted into lethally irradiated mice and long-term multilineage reconstitution was analyzed up to 16 weeks later. Long-term multilineage reconstitution was again restricted to the TMRM^{low} subpopulation **(****Fig. 3C****).** Moreover, secondary transplantation showed long-term engraftment of the TMRM^{low} fraction **(****Fig. 3C****).** Most importantly, these data show that a low ΔΨ m is not only a hallmark of quiescent LT- HSC *in vivo* but also of activated stem cells that have undergone a self-renewing cell division. To our knowledge, this is the first read-out for self-renewal divisions of HSCs *in vitro* where the commonly used cell surface repertoire fails to identify such cells, as also demonstrated.

### HSC culture

HSCs were cultured under 'expansion' conditions in U-bottom 96-well plates for five days. Cultures were maintained in Stemline II (Sigma) supplemented with 10 µg/ml Heparin (Sigma), 100 ng/ml SCF (R&D Systems), 2 ng/ml Flt3 ligand (R&D), 20 ng/ml TPO (R&D Systems), 10 ng/ml FGF-1 (Invitrogen), 500 ng/ml IGFBP2 (R&D Systems), 100 ng/ml AngL-3 (R&D Systems). At the end of the culture period cells were stained with TMRM and analyzed or sorted by flow cytometry. To induce differentiation, HSCs were cultured in basal medium (Stemline II containing 100 ng/ml SCF and 2 ng/ml Flt3 ligand) supplemented with 20 ng/ml IL-3 (R&D Systems) and 100 ng/ml IL-6 (R&D Systems).

### CFSE staining

Freshly sorted LT-HSCs were incubated for 20 min at 37°C with 1:400 CFSE stock solution (Cayman chemicals; CFSE cell division assay kit). The cells were pelleted and re-suspended in 1 ml of Stemline II (Sigma) containing 10% FBS for 20 min at 37°C. Thereafter, the cells were washed twice with 1 ml of Stemline II (Sigma) and put in culture.

### Example 3: Preservation of HSC self-renewal under differentiation conditions

The modulation of HSC fate through the modulation a cell's mitochondrial activity level was investigated. Specifically, culture conditions that would rapidly differentiate HSCs into highly proliferative MPPs were chosen and the effect of blocking the establishment of ΔΨm on the maintenance of stemness was investigated. For that purpose, trifluorocarbonylcyanide phenylhydrazone (FCCP) which permeabilizes the inner mitochondrial membrane and disrupts its potential was used as an uncoupling agent of the electron transport from ATP generation. Indeed, flow cytometry analysis showed that the disruption of ΔΨm by FCCP resulted in decreased NADH levels in cultured HSCs.
In a first series of experiments, quantitative clonal differentiation assays (short- and long-term colony forming unit (CFU) assays as described above) were used to test to which extent mitochondrial uncoupling could influence the colony forming behavior and efficiency of hematopoietic stem and progenitor cells **(****Fig. 4A-C****).** 15'000 whole bone marrow cells cultured under differentiation conditions for two days in the presence of FCCP showed a striking decrease in the total number of colonies, as well as an increase in the percentage of clonogenic multi-lineage colonies (CFU-GEMMs: colony forming unit-granulocyte, erythrocyte, macrophage, megakaryocyte) which originate only from the most primitive progenitor cells **(****Fig. 4A****,** right panel). In contrast, the total number of colonies and the percentage of colonies derived from more committed progenitors (e.g. macrophage colonies, M, granulocyte/macrophage colonies, GM) were markedly decreased **(****Fig. 4A****,** left and middle panel, respectively), suggesting that FCCP might slow down colony formation by enforcing self-renewal over differentiation. Similarly, when 100 LT-HSC:TMRM^{low} cells were first cultured for two days under differentiation conditions in the presence or absence of FCCP **(****Fig. 4B****),** a striking decrease in the frequency of colonies derived from the more committed progenitors (Mk, G, M and GM) **(****Fig. 4B****,** left and middle panel) and a concomitant increase of the ones derived from the most primitive cells (CFU-GEMM) when cultured with FCCP **(****Fig. 4B****,** right panel) was observed. These experiments strongly suggested that mitochondrial uncoupling during differentiation-induction skews HSC fate from rapid commitment towards self-renewal. Photographs of each experimental condition (+/- FCCP) reveal huge GEMM colonies (white) that are visible by eye in the case of mitochondrial uncoupling.
To further corroborate these results, long-term blood reconstitution assays wherein LT-HSC:TMRM^{low} were cultured for five days under differentiation condition (SCF, Flt3, IL-3 and IL-6) as described above in the presence or absence of FCCP, and transplanted all progeny into lethally irradiated mice together with 2*10⁶ helper cells as described below. Strikingly, FCCP-exposed cells showed dramatically higher levels of long-term multilineage blood reconstitution in recipient mice **(****Fig. 4C****).** Importantly, by labeling HSCs with CFSE and tracking their divisional history, it could be ruled out that this effect is due to an induction of quiescence upon FCCP administration, as all cells in culture are rapidly dividing several times. Therefore, by blocking OXPHOS, HSCs that would normally rapidly differentiate can be forced to undergo self-renewal divisions. In line with the *in vivo* data above, these data show that self-renewal divisions of HSCs do not require OXPHOS and may thus be executed by the cells via mitochondria-independent processes.
Altogether those data show that each phenotypically defined hematopoietic stem and progenitor cell compartment has a very distinct ΔΨm level. *In vivo* multi-lineage blood reconstitution assays show that within each population, functional short-term and long-term self-renewal activity is precisely restricted to subpopulations having lower ΔΨm. Intriguingly, comparison of ΔΨm levels of HSCs separated by their cell cycle phase, shows that during homeostasis and as well as under acute stress, quiescent and cycling HSC have similar metabolic profiles based on their ΔΨm levels. This shows that distinct metabolic program of HSCs is not restricted to their quiescent state but rather indicative of their fate choice. Indeed, *in vitro,* in heterogeneous HSC expansion cultures, divisional tracking assays show that self-renewing HSCs can be recognized by their low ΔΨm, in marked contrast to differentiated cells that have activated mitochondria. Strikingly, chemical uncoupling of the electron transport chain enforces HSC self-renewal even under culture conditions that normally induced rapid differentiation.
Therefore, not only it is shown that low mitochondrial membrane potential ΔΨm is a potent functional marker of HSCs, but also it is here revealed that stem cell fate can be manipulated by acting on this parameter for providing avenues for HSC culture and therapies.

### Transplantation of HSC cultured exposed to the uncoupler FCCP

100 LT-HSC:TMRM^{low} cells were cultured for five days under differentiation-inducing conditions in the presence or absence of 5 µM FCCP. Cell progeny were transplanted in lethally irradiated recipient mice together with 2*10⁶ helper cells. Peripheral blood was collected at 4, 8 and 12 and 16 weeks to determine the level of chimerism as described above.

### HSC culture

For some experiments, 5 or 10 µM Carbonyl cyanide 4-(trifluoromethoxy) phenylhydrazone (FCCP) (Sigma) was added at the medium. FCCP stock solution was prepared by dissolving the powder in ethanol at 10 mM concentration.

All these data support a novel approach of predicting and directing hematopoietic stem cell fate based on mitochondrial metabolism. In fact, they show that the known surface marker panels commonly used to purify HSCs and their offspring isolate highly heterogeneous cell populations with different stem cell potential which is problematic for studying mechanisms of stem cell fate and for improving strategies for the clinical expansion of these rare cells.
By adding a mitochondrial activity-based read-out, namely mitochondrial membrane potential ΔΨm, to the existing surface marker repertoire, those data show that it is possible to enrich stem cell pools in functional stem cells and to detected a cell population (LT-HSC:TMRM^{high)} that lacks multi-lineage reconstitution. Therefore, ΔΨm serves as robust prospective marker for HSC long-term multilineage blood reconstitution and also marks live, self-renewing HSCs. Moreover, modulation of mitochondrial activity by treatment with an uncoupling agent, such as FCCP, can redirect HSCs fate *in vitro* under differentiation conditions. These results provide evidence for the pivotal role of mitochondrial metabolism in HSC fate determination and suggest novel avenues for the ex-vivo expansion and the therapeutic manipulation of HSC fate via mitochondria metabolism.

### Example 4: Effect of a mitochondrial membrane potential reducing agent on expansion of hematopoietic progenitor compartments in mouse bone marrow

Short-term (1 week) and long-term (4 weeks) treatments with an agent able to reduce mitochondrial membrane potential, NR, were tested in mice as described below on the hematopoietic stem and progenitor pool sizes in the bone marrow.
Flow cytometry analyses of the BM samples showed an expansion of the hematopoietic progenitor compartments both in short- and long-term treatments (Fig. 5A). The increased cellularity is observed in all hematopoietic progenitor compartments after 1 week of treatment, whereas after 4 weeks treatment only the short-term compartment (ST-HSC) is expanded. Surprisingly, progenitor expansion was not accompanied by a reduction of the HSC pool (Fig. 5A, B). This could indicate that the effect of NR was restricted to hematopoietic progenitor cells, or that HSC fate was affected by promoting higher levels of asymmetric self-renewal divisions that would not alter overall stem cell numbers.
BM derived from 1-month NR-treated mice do not show engraftment advantage as evaluated by transplantation assays as described previously (Vannini et al., 2012, Cell Cycle, 211(8):1535-43*),* indicating that NR does not affect the stem cell compartment directly. Therefore, NR-treatment is not expected to exhaust the LT-HSC population, which is often the problem when increasing short-term HSCs and their progeny. Colony-forming functional assays (termed 'CFUs', was performed accordingly to manufacturer instructions (Methocult™, Stem Cell technology) and confirmed the expansion of hematopoietic progenitor compartments, especially in the BM of short-term treated mice (Fig. 5C). The colony increase was most striking in the Mk lineage.

### Example 5: Effect of a mitochondrial membrane potential reducing agent, Nicotinic Acid (NA) on expansion of hematopoietic progenitor compartments in mouse bone marrow

Short-term (1 week) treatments with NA (A, B), another agent able to reduce mitochondrial membrane potential in HSCs, was tested in mice as described above to measure changes in the hematopoietic stem and progenitor pool sizes in the bone marrow. Flow cytometry analyses of the BM samples showed an expansion of the LT-HSC compartments for both agents (Fig. 6A1-A3). Cell cycle analysis did not reveal any significant difference between treated and non-treated conditions for NA (Fig. 6B1-B2).

### Example 6: Effect of a decrease of mitochondrial membrane potential on survival and blood recovery in mice after transplantation (limiting-dose transplanted mice)

Given the dependency of differentiated hematopoietic cells to the progenitor compartments, the effect of a mitochondrial membrane potential reducing agent according to the invention, in particular NR, in improving blood recovery and survival of transplanted mice was assessed. The timelines of the protocol are shown in Fig 7A1. Peripheral blood count started at day 13 after transplantation, and it was repeated every 3 or 4 days.
Strikingly, 80% of NR-treated mice (square) survive whereas all untreated mice (dot) died within one month (Fig. 7A1). Furthermore, NR-treated mice had faster neutrophil and platelet recovery (Fig. 7B1 and 7B2).
The exit of neutropenia is a critical factor for a positive patient outcome, as neutrophils constitute the first barrier against infections in patients is the most important determinant to allow post-transplanted patients to be discharged from hospital. Mice treated with NR exited neutropenia (neutrophils > 0.5 G/l), on average, one week before controls (Fig. 7B2). Additionally, recovery from severe thrombocytopenia (platelet count > 100 G/l) occurs on average 10 days earlier in NR-treated mice relative to control mice (Fig. 7B1).

Therefore, NR treatment improves survival and blood recovering in a context of transplantation.

### Example 7: Effect of Nicotinamide Riboside on mitochondrial activity and division synchrony of HSCs in vitro

The effects of NR on HSC mitochondrial activity has been assessed by measuring mitochondrial activity using JC1 *(*Mantel et al., 2010, Cell Cycle, 9(10), 2008-17) and TMRM dyes *(Vannini et al., 2014, in press).* HSCs exposed to NR have a robust decrease in mitochondrial activity (Fig. 8A).

Also in dividing HSCs, NR raised the proportion of cells with low mitochondrial activity (Fig. 8B1-B2), indicating an increase of self-renewing activity. Furthermore, at single cell level the effect of NR on cell proliferation kinetics was determined as described previously *(Vannini et al., 2012, supra).* HSCs exposed to NR do not have a major change in their proliferation kinetics, but they display a significant increase in asynchronous division (Fig. 8C).
These results suggest the possibility that NR pushed HSC fate toward asymmetric division.
Taken together those results indicate that treatment with a mitochondrial membrane potential reducing agent according to the invention, such as NR, treatment, through the food leads to the expansion of hematopoietic progenitor pools in the bone marrow and, when tested in a limiting cell dose transplantation scheme, results in a dramatic improvement of survival. Importantly, the HSC pool size was not affected by NR administration, suggesting that the risk of stem cell exhaustion in such a treatment is minimal.
Therefore, those data support that treatment with a mitochondrial membrane potential reducing agent according to the invention, such as NR, would be useful for enhancing blood recovery and maintaining HSCs pool of transplanted patients or those receiving ablative chemotherapy for solid tumor or severe immunological disorders.

### Mice

C57B1/6J and C57B1/6J Ly5.1 were purchased from Charles River Laboratories International and maintained in micro-isolator cages. Mice were provided continuously with sterile food, water and bedding. Nicotinamide riboside (NR) (custom synthesized as triflate salt by Novalix in Strasbourg) was supplied in the food at 0.4 g/Kg mouse and mice were treated for 1 and 4 weeks respectively.

### Survival and blood recovery

C57B1/6 Ly5.2 mice were lethally irradiated at 850 RAD and transplanted with total BM donor cells derived from C57B1/6 Ly5.1 mice (70*10³ and 150*10³). Mice were followed daily to assess their general health state. Peripheral blood was analyzed for leukocyte and erythrocyte recovery every three days starting at 13 days after transplant as shown on the protocol time lines on Figure 2A. For transplants of HSC cultured exposed to the uncoupler FCCP, 100 LT-HSC:TMRM^{low} cells were cultured for five days under differentiation-inducing conditions in the presence or absence of 5 µM FCCP. Cell progeny were transplanted in lethally irradiated recipient mice together with 2*10⁶ helper cells. Peripheral blood was collected at 4, 8 and 12 weeks to determine the level of chimerism.

### Analysis of single cell cycle kinetics

To follow the behavior of HSCs at single cell level, cells were sorted directly into 96-well plates coated with microwell arrays. Microwells containing a single cell at the time of plating were tracked by time-lapse microscopy.Proliferation kinetics were assessed using a Zeiss Axio Observer microscope equipped with an incubator chamber, temperature and CO₂ control. Images were automatically acquired every three hours over five days using the imaging analysis software of MetaMorph (Visitron, Germany).

### Hydrogel microwell array production

Hydrogel microwell arrays were directly casted within individual wells of a 96-well plate as described *(*Kobel et al., 2009, Langmuir, 25(15):8774-9*;* Lutolf et al., 2009, Integr Biol (Camb), 1(1):59-69*).* Briefly, stoichiometrically balanced aqueous solutions of multi-arm poly(ethylene glycol) (PEG), end-functionalized with thiol and vinylsulfone groups were mixed and molded against a PDMS microstamp. Upon completion of crosslinking, the stamp was removed and the hydrogel microwell array was hydrated overnight at 4 °C and then sterilized with ultraviolet light.

### Statistics

Data were statistically analyzed by Student's t test, one way ANOVA followed by Bonferroni's multiple comparison test and Mann Whitney test.

## Claims

1. A mitochondrial membrane potential reducing agent for use in the prevention and/or treatment of a decreased blood cell level as compared to a control blood cell level.

2. A mitochondrial membrane potential reducing agent for use according to claim 1 wherein the decreased blood cell level is severe neutropenia and/or severe thrombocytopenia.

3. A mitochondrial membrane potential reducing agent for use according to claim 1 or 2 wherein said mitochondrial membrane potential reducing agent it to be administered orally.

4. A mitochondrial membrane potential reducing agent for use according to any one of claims 1 to 3, wherein said mitochondrial membrane potential reducing agent is to be administered parenterally.

5. A mitochondrial membrane potential reducing agent for use according to any one of claims 1 to 4 wherein the said agent is selected from nicotinamide riboside, niacin, nicotinic acid, N-formylkynurenine, quinolinic acid, nictotinamide riboside kinase activator, nicotinamide mononucleotide, acipimox and tryptophan.

6. A mitochondrial membrane potential reducing agent for use according to any one of claims 1 to 5 wherein the said agent is nicotinamide riboside or nicotinic acid.

7. A method for *ex-vivo* hematopoietic stem cell expansion comprising the steps of:
a) providing a HSC sample in a cell expansion culture medium;
b) optionally isolating HSC fraction **characterized by** a low mitochondrial membrane potential from said HSC sample into an isolated HSC pool;
c) contacting HSC sample provided under a) or HSC pool isolated under b) with a mitochondrial membrane potential reducing agent.

8. A method according to claim 7, wherein the HSC sample obtained under step c) is enriched in functional stem cells having long-term multi-lineage blood reconstitution capability.

9. A method according to claim 7 or 8, wherein the HSC sample is a bone marrow sample and/or bone marrow cell preparation.

10. A method according to any one of claims 7 to 9 wherein the mitochondrial membrane potential reducing agent is selected from the following group: 2,4 di-nitrophenol, Carbonyl cyanide 4-(trifluoromethoxy)phenylhydrazone, Carbonyl cyanide m-chloro phenyl hydrazine, 2-fluorophenyl){6-[(2-fluorophenyl)amino](1,2,5-oxadiazolo[3,4-e]pyrazin-5-yl)}amine and 4,5,6,7-Tetrachloro-2-trifluoromethylbenzimidazole.

11. A method according to any one of claims 5 to 10, wherein the mitochondrial membrane potential reducing agent is carbonyl cyanide 4-(trifluoromethoxy)phenylhydrazone.

12. A method according to any one of claims 7 to 10, wherein the mitochondrial membrane potential reducing agent is selected from nicotinamide riboside, niacin, nicotinic acid, N-formylkynurenine, quinolinic acid, nictotinamide riboside kinase activator, nicotinamide mononucleotide, acipimox and tryptophan.

13. A kit for selecting functional stem cells having long-term multi-lineage blood reconstitution capability comprising said kit comprising i) at least one detecting agent for surface marker for phenotypically defined hematopoietic stem and progenitor cell or a combination of several of those markers and ii) at least one agent for detecting low mitochondrial membrane potential in hematopoietic stem and progenitor cells, such as JC1 or TMRM dye.

14. A kit according to claim 13 wherein the said at least one detecting agent for phenotypically defined hematopoietic stem and progenitor cell is one or more selective antibody directed to said surface marker selected from c-Kit, Lin-, Sca-1, CD150, CD48 and CD34.

15. A cell expansion culture medium for hematopoietic stem cell preparations comprising a mitochondrial membrane potential reducing agent.
